# EUROPÄISCHE PATENTANMELDUNG

(11) **EP 2 962 659 A1**
(43) Veröffentlichungstag der Anmeldung: **06.01.2016**
(21) Anmeldenummer: 14175369.9
(22) Anmeldetag: 02.07.2014
(51) Int. Cl.: A61C 19/00, A61B 19/00

(54) **Reinigungs- und/ oder Pflegegerät für zumindest ein medizinisches, insbesondere zahnärztliches, Instrument**

(71) Anmelder: W & H Dentalwerk Bürmoos GmbH, 5111 Bürmoos (AT)
(72) Erfinder: Tannebaum, Wolfgang, 92637 Weiden (DE)

(57) **Zusammenfassung**

Reinigung- und/ oder Pflegegerät (1) für zumindest ein medizinisches, insbesondere zahnärztliches, Instrument (2) mit zumindest einer Medienquelle (3) und/ oder zumindest einem Medienanschluss zur Versorgung des Reinigungs- und/ oder Pflegegeräts (1) mit zumindest einem Arbeitsmedium, insbesondere mit einem Antriebsfluid, zumindest einer Kupplungsvorrichtung (4, 4', 4") zur lösbaren Verbindung des medizinischen, insbesondere zahnärztlichen, Instruments (2) mit dem Reinigungs-und/ oder Pflegegerät (1), sowie zumindest einer Medienzuführung (5), welche die zumindest eine Medienquelle (3) und/ oder den zumindest einen Medienanschluss mit dem zumindest einen zu reinigenden und/ oder pflegenden medizinischen Instrument (2) verbindet, wobei die zumindest eine Medienzuführung (5) zumindest eine Aufnahmevorrichtung (6, 6', 6") für zumindest eine Reinigungs- und/ oder Pflegekapsel (7, 7', 7") aufweist, wobei die Aufnahmevorrichtung (6, 6', 6") derart ausgebildet ist, dass die zumindest eine Reinigungs- und/ oder Pflegekapsel (7, 7', 7") von dem zumindest einen Arbeitsmedium der Medienquelle (3) und/ oder des Medienanschlusses durchströmbar ist, so dass das Arbeitsmedium mit dem Reinigungs- und/ oder Pflegemedium in das zu reinigende und/ oder pflegende Instrument (2) einbringbar ist.

## Beschreibung

Die vorliegende Erfindung bezieht sich auf ein Reinigungs- und/ oder Pflegegerät für zumindest ein medizinisches, insbesondere zahnärztliches, Instrument nach dem Oberbegriff des Anspruchs 1, auf eine Reinigungs- und/ oder Pflegekapsel nach Anspruch 11, sowie auf ein Verfahren zum Reinigen und/ oder Pflegen zumindest eines medizinischen, insbesondere zahnärztlichen, Instruments nach dem Oberbegriff des Anspruchs 15.

Derartige Reinigungs- und/ oder Pflegegeräte dienen zum Reinigen, Desinfizieren, Sterilisieren und/ oder Pflegen von medizinischen, insbesondere zahnärztlichen, Instrumenten. Als zu reinigende Instrumente werden insbesondere gerade, gebogene, oder pistolenförmige Handstücke als auch Teile von Handstücken, z. B. Handstückköpfe mit einer Werkzeugaufnahme zur Aufnahme eines Behandlungswerkzeugs, Adapter und Kupplungen verstanden. Durch die Handstücke erstrecken sich oftmals Versorgungsleitungen zum Antrieb eines Behandlungswerkzeugs sowie Fluidleitungen. Bei den Leitungen handelt es sich insbesondere um Getriebekanäle oder Fluidkanäle für Luft, Wasser oder Spray.

Nach dem Betrieb der Instrumente oder vor der erstmaligen Benutzung bedarf es in regelmäßigen Abständen einer Reinigung und/ oder Pflege der Leitungen, insbesondere der Antriebskanäle sowie der Fluidkanäle. Hierzu stehen dem Anwender eine Vielzahl von Reinigung- und Pflegegeräte zu Verfügung. Diese Geräte weisen in einem Reinigungs- und/ oder Pflegebereich zumindest eine Kupplungsvorrichtung zur Reinigung der im Inneren der Instrumente angeordneten Versorgungs- und Fluidleitungen auf. Die medizinischen Instrumente werden hierbei bevorzugt in einer etwa vertikalen Reinigungsstellung in der Reinigungskammer aufgenommen, so dass die überschüssigen Reinigungs- und Pflegemittel aus dem Instrument heraus rinnen können.

Ein derartiges Reinigungs- und/ oder Pflegegerät ist insbesondere aus der DE 10 2010 002 030 A1 bekannt.

Dieses bekannte Reinigungsgerät weist ein Gehäuse mit einer Reinigungskammer für zumindest ein medizinisches Instrument auf, wobei ein Verbindungsteil zur Aufnahme des einen Ende des Instruments in der Reinigungskammer angeordnet ist. Reinigungs- und/ oder Pflegemedien werden in Vorratsbehältern bereitgestellt. Zur Förderung der Reinigungs- und/ oder Pflegemedien von den Behältern in die Instrumente und/ oder in die Reinigungskammer werden entweder eine oder mehrere Förderpumpen in den jeweiligen Medienzuleitungen oder zumindest eine Vakuumpumpe in der Reinigungskammer eingesetzt. Die Fluide werden hierbei aus den Behältern in die medizinische Instrumente und/ oder in die Reinigungskammer gepumpt bzw. gesaugt.

Als nachteilig dieser Ausgestaltung des Reinigungsgeräts erweist sich die Bereitstellung der Reinigungs- und/ oder Pflegemedien in Vorratsbehältern, welche dazu ausgebildet sind die Medien in einer ausreichend großen Menge aufzubewahren, um eine Vielzahl von Instrumente zu reinigen und/ oder zu pflegen. Die Aufnahme der Medien in den Vorratsbehältern ermöglicht zwar eine Aufbereitung einer Vielzahl von Instrumenten, jedoch ist eine individuelle Reinigung und/ oder Pflege unterschiedlicher Instrumente, insbesondere von unterschiedlichen Herstellern, nach deren Vorgaben, insbesondere mit deren Reinigungs- und/ oder Pflegemedien, mittels der bekannten Vorrichtung nicht möglich. Die bekannte Vorrichtung stellt mittels der Vorratsbehälter jeweils lediglich ein einziges Reinigungs- und/ oder Pflegemedium zur Verfügung. Um eine individuelle Reinigung- und/ oder Pflege der Instrumente mit der bekannten Vorrichtung zu ermöglichen, müssten die mehreren Vorratsbehälter nach jedem Reinigungs- und/ oder Pflegezyklus eines Instruments getauscht werden. Des Weiteren müssten dem Anwender eine Vielzahl von Vorratsbehältern zur Verfügung gestellt werden.

Einen weiteren Nachteil der im Stand der Technik bekannten Ausführungsform stellt die bekannte Medienzuführung dar, um die Reinigungs- und/ oder Pflegemedien aus den Vorratsbehältern in die Instrumente zu fördern. Durch die Aufbewahrung der Medien in Vorratsbehältern ist eine aufwendige Medienzuführung mit einer Vielzahl von Leitungen, Ventilen und Pumpen erforderlich. Hierdurch besteht die Gefahr einer möglichen Prozessstörung auf Grund möglicher Ablagerungen von Prozessmedien in den mehreren Leitungen.

Des Weiteren ist auf Grund der geringen Mengen von Reinigungs- und/ oder Pflegemedien, welche zur Aufbereitung eines einzigen Instruments benötigt werden, ein erheblicher technischer Aufwand notwendig, um diese präzise, insbesondere in ausreichend genauer Quantität, in die aufzubereitenden Instrumente dosieren zu können.

Der vorliegenden Erfindung liegt daher die Aufgabe zugrunde ein Reinigungs- oder Pflegegerät zu schaffen, welches bei einfacher Herstellung die Nachteile des Stand der Technik vermeidet und es insbesondere ermöglicht medizinische, insbesondere zahnärztliche, Instrumente bedienungsfreundlich und individuell nach Angaben der Hersteller zu reinigen und/ oder zu pflegen.

Zur Lösung dieser Aufgabe weist das erfindungsgemäße Reinigung- und/ oder Pflegegerät für zumindest ein medizinisches, insbesondere zahnärztliches, Instrument zumindest eine Medienquelle und/ oder zumindest einen Medienanschluss zur Versorgung des Reinigungs- und/ oder Pflegegeräts mit zumindest einem Arbeitsmedium, insbesondere mit einem Antriebsfluid, zumindest eine Kupplungsvorrichtung zur lösbaren Verbindung des medizinischen, insbesondere zahnärztlichen, Instruments mit dem Reinigungs- und/ oder Pflegegerät, sowie zumindest eine Medienzuführung auf, welche die zumindest eine Medienquelle und/ oder den zumindest einen Medienanschluss mit dem zumindest einen zu reinigenden und/ oder pflegenden medizinischen Instrument verbindet, wobei die zumindest eine Medienzuführung zumindest eine Aufnahmevorrichtung für zumindest eine Reinigungs- und/ oder Pflegekapsel aufweist, wobei die Aufnahmevorrichtung derart ausgebildet ist, dass die zumindest eine Reinigungs- und/ oder Pflegekapsel von dem zumindest einen Arbeitsmedium der Medienquelle und/ oder des Medienanschlusses durchströmbar ist, so dass das Arbeitsmedium mit dem Reinigungs- und/ oder Pflegemedium in das zu reinigende und/ oder pflegende Instrument einbringbar ist.

Gemäß einem bevorzugten Ausführungsbeispiel weist die zumindest eine Aufnahmevorrichtung der Medienzuführung zumindest einen Medieneinlass und zumindest einen Medienauslass auf, welche mit zumindest einem Medieneinlass und zumindest einem Medienauslass der Reinigungs- und/ oder Pflegekapsel koppelbar sind.

Des Weiteren umfasst die Aufnahmevorrichtung bevorzugt einen Betätigungsmechanismus, um den zumindest einen Medieneinlass und den zumindest einen Medienauslass der Reinigungs- und/ oder Pflegekapsel mit dem zumindest einen Medieneinlass und dem zumindest einen Medienauslass der Aufnahmevorrichtung zu verbinden. Der Betätigungsmechanismus ist hierzu bevorzugt durch ein erstes feststehendes Gehäuseteil und durch ein zweites zu dem ersten Gehäuseteil beweglich gelagerten Gehäuseteil gebildet, wobei eines der Gehäuseteile den zumindest einen Medieneinlass und das andere Gehäuseteil den zumindest einen Medienauslass der Aufnahmevorrichtung umfasst.

Gemäß einem weiteren Ausführungsbeispiel des Reinigung- und/ oder Pflegegeräts ist die zumindest eine Aufnahmevorrichtung mit zumindest einer Medienleitung verbunden, welche derart in dem Reinigungs- und/ oder Pflegegerät angeordnet ist, dass das Arbeitsmedium mit dem Reinigungs- und/ oder Pflegemedium auf der Außenseite des zu reinigenden und/ oder pflegenden Instruments aufbringbar ist.

Des Weiteren ist die zumindest eine Kupplungsvorrichtung bevorzugt drehbar und vorzugsweise lösbar an dem Reinigungs- und/ oder Pflegegerät angeordnet. Alternativ kann auch die Aufnahmevorrichtung für die Reinigungs- und/ oder Pflegekapsel drehbar an dem Reinigungs- und/ oder Pflegegerät ausgebildet sein.

Gemäß einem weiteren Ausführungsbeispiel umfasst die zumindest eine Kupplungsvorrichtung ein Gehäuse, welches das zumindest eine zu reinigende und/ oder pflegende Instrument, vorzugsweise druckdicht, umgibt.

Gemäß einem weiteren Ausführungsbeispiel des Reinigung- und/ oder Pflegegeräts weist die zumindest eine Medienzuführung und/ oder die zumindest eine Aufnahmevorrichtung zum Erwärmen des Arbeitsmediums und/ oder des Reinigungs- und/ oder Pflegemediums zumindest ein Heizelement auf. Zum Fördern und/ oder Verdichten des zumindest einen Arbeitsmediums und/ oder des zumindest einen Reinigungs- und/ oder Pflegemediums umfasst die zumindest eine Medienzuführung und/ oder die zumindest eine Aufnahmevorrichtung des Weiteren bevorzugt zumindest eine Pumpen- und/ oder Kompressoreinheit.

Gemäß einem weiteren Ausführungsbeispiel des Reinigung- und/ oder Pflegegeräts weist die zumindest eine Aufnahmevorrichtung eine Erkennungsvorrichtung auf, welche ausgebildet ist ein an der zumindest einen Reinigungs- und/ oder Pflegekapsel angebrachtes Identifizierungselement zu erkennen, so dass die für das jeweilige Prozessergebnis relevanten Betriebsparamater, insbesondere jene des zumindest einen Heizelements und/ oder der zumindest einen Pumpen- und/ oder Kompressoreinheit, mittels einer Steuer- und/ oder Bedieneinheit automatisch einstellbar sind.

Gemäß einem Ausführungsbeispiel einer Reinigungs- und/ oder Pflegekapsel für das Reinigungs- und/ oder Pflegegerät weist diese ein Gehäuse mit zumindest einer Kammer zur Aufbewahrung von zumindest einem Reinigungs- und/ oder Pflegemedium, zumindest einen versiegelten Medieneinlass sowie zumindest einen versiegelten Medienauslass auf, wobei die zumindest eine Kammer ausgebildet ist mindestens 0,05 Milliliter und maximal 5 Milliliter Reinigungs- und/ oder Pflegemedium zu speichern, so dass mittels der Reinigungs- und/ oder Pflegekapsel ein medizinisches Instrument reinigbar und/ oder pflegbar ist.

Gemäß einem weiteren Ausführungsbeispiel der Reinigungs- und/ oder Pflegekapsel umfasst das Gehäuse der Reinigungs- und/ oder Pflegekapsel zumindest eine zweite Kammer für ein zweites Reinigungs- und/ oder Pflegemedium, wobei die zweite Kammer ausgebildet ist mindestens 20 Milliliter und maximal 100 Milliliter Reinigungs- und/ oder Pflegemedium zu speichern.

Des Weiteren weist die Reinigungs- und/ oder Pflegekapsel bevorzugt ein Identifizierungselement, insbesondere einen Strichcode, zur Erkennung der Reinigungs- und/ oder Pflegekapsel auf.

Gemäß einem Ausführungsbeispiel eines Verfahrens zum Reinigen und/ oder Pflegen zumindest eines medizinischen, insbesondere zahnärztlichen, Instruments mit einem Reinigungs- und/ oder Pflegegerät wird ein Arbeitsmedium, insbesondere ein Antriebsfluid, mittels einer Medienzuführung von einer Medienquelle und/ oder einem Medienanschluss durch eine Aufnahmevorrichtung für eine Reinigungs- und/ oder Pflegekapsel, welche zumindest ein Reinigungs- und/ oder Pflegemedium aufweist, in eine Kupplungsvorrichtung für das zumindest eine medizinische, insbesondere zahnärztliche, Instrument geleitet, wobei die in der Aufnahmevorrichtung angeordnete Reinigungs- und/ oder Pflegekapsel von dem Arbeitsmedium durchströmt wird und das Reinigungs- und/ oder Pflegemedium mit dem Arbeitsmedium in das zu reinigende und/ oder pflegende Instrument geleitet wird.

Bevorzugt wird hierbei das Arbeitsmedium und/ oder das Reinigungs- und/ oder Pflegemedium mittels eines Heizelements erwärmt und/ oder mittels einer Pumpen- und/ oder Kompressoreinheit verdichtet. Alternativ ist es auch möglich, dass das Arbeitsmedium in einem unter Druck stehenden Behälter bereits gestellt wird und in die Medienzuführung, insbesondere in die Aufnahmevorrichtung und in die Reinigungs- und/ oder Pflegekapsel mittels eines Treibmittels gedrückt wird.

Das vorliegende Reinigungs- und/ oder Pflegegerät zeichnet sich durch eine Reihe erheblicher Vorteile aus.

Durch die erfindungsgemäße Ausgestaltung des Reinigungs- und/ oder Pflegegeräts wird eine individuelle Reinigung und/ oder Pflege medizinischer, insbesondere zahnärztlicher, Instrumente ermöglicht. Je nach Instrumententyp und/ oder Hersteller können Reinigungs- und/ oder Pflegemedien mittels der zugehörigen Reinigungs- und/ oder Pflegekapsel in dem Reinigungs- und/ oder Pflegegerät zur Verfügung gestellt werden. Die medizinischen Instrumente können somit in einfacher Weise nach Herstellervorgaben gereinigt und/ oder gepflegt werden.

Einen weiteren Vorteil der Erfindung bildet die kostengünstige Fertigung des Reinigungs- und/ oder Pflegegeräts. Durch die Aufnahme der bevorzugt mehreren Reinigungs- und/ oder Pflegemedien für ein zu reinigendes und/ oder pflegendes Instrument in einer Reinigungs- und/ oder Pflegekapsel, ist lediglich eine Aufnahmevorrichtung für diese Kapsel notwendig. Die Ausbildung und das zur Verfügung stellen mehrerer Vorratsbehälter und Aufnahmevorrichtungen für diese ist nicht mehr notwendig.

Des Weiteren ermöglicht auch die vereinfachte Ausführung der Medienzuführung eine Reduzierung der Herstellkosten für das Reinigungs- und/ oder Pflegegerät. Eine komplexe Medienzuführung zur Verbindung der medizinischen Instrumente mit den mehreren Vorratsbehältern, wie aus dem Stand der Technik bekannt, mit mehreren Ventilen, Pumpen sowie verzweigten Medienleitungen ist ebenso nicht mehr notwendig.

Einen weiteren Vorteil des Reinigungs- und/ oder Pflegegeräts bildet die Identifizierung der zu reinigenden bzw. zu pflegenden Instrumente durch die Reinigungs- und/ oder Pflegekapsel. Durch die Erkennungsvorrichtung in dem Reinigungs- und/ oder Pflegegerät, welche ausgebildet ist ein an der zumindest einen Reinigungs- und/ oder Pflegekapsel angebrachtes Identifizierungselement zu erkennen, ist es möglich Betriebsparamater für das zumindest eine Reinigungs- und/ oder Pflegemedium oder für das Antriebsfluid, wie zum Beispiel Temperatur, Druck oder Zeit, abhängig von dem zu reinigenden und/ oder pflegenden Instrument automatisch einzustellen.

Nachfolgend wird die Erfindung anhand mehrerer Ausführungsbeispiele und in Verbindung mit den beigefügten Zeichnungen erläutert.

Dabei zeigen:
Figur 1 ein Ausführungsbeispiel des Reinigungs- und/ oder Pflegegeräts in der Außenansicht,
Figur 2 eine schematische Darstellung des Reinigungs- und/ oder Pflegegeräts, insbesondere einen Fluidplan des Reinigungs- und/ oder Pflegegeräts,
Figur 3 ein Ausführungsbeispiel der Reinigungs- und/ oder Pflegekapsel in der Seitenansicht,
die Figuren 4A bis 4C drei Ausführungsbeispiele der Aufnahmevorrichtung und Kupplungsvorrichtung des Reinigungs- und/ oder Pflegegeräts sowie der Reinigungs- und/ oder Pflegekapsel;

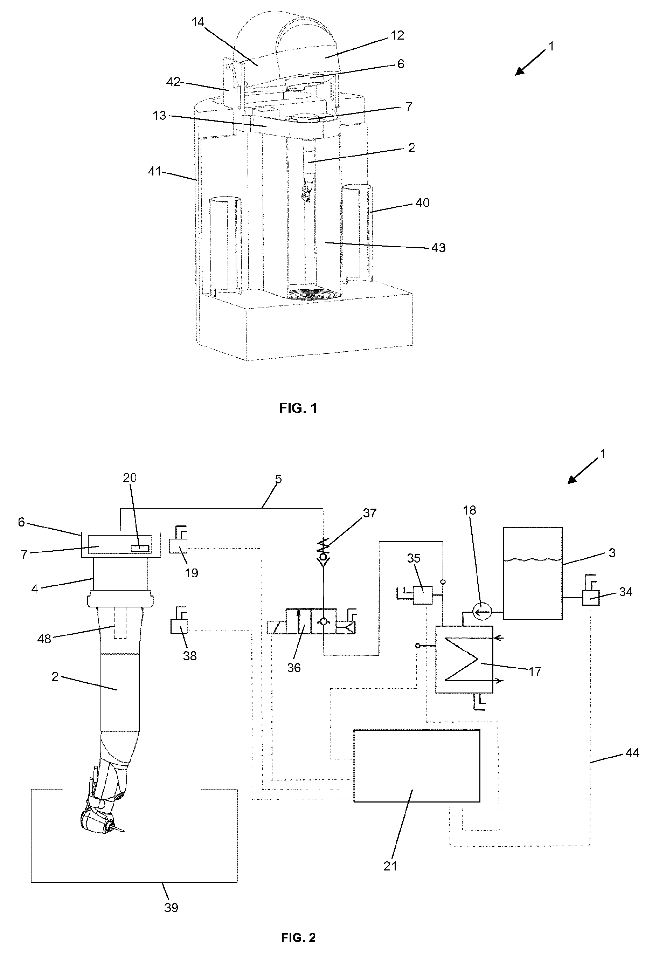

Das in Figur 1 dargestellte Reinigungs- und/ oder Pflegegerät 1 für zumindest ein medizinisches, insbesondere zahnärztliches, Instrument 2 umfasst ein Gehäuse 41 mit mehreren Außenwänden, eine Ablage 40 für eine oder mehrere Reinigungs- und/ oder Pflegekapseln 7, sowie einen Aufnahmebereich 43 für das zu reinigende und/ oder pflegende Instrument 2. In dem Gehäuse 2 sind neben einer Steuer- und/ oder Bedieneinheit weitere für den Reinigungs- oder Pflegeprozess notwendige Bauteile, insbesondere eine Medienzuführung, wie in Figur 2 gezeigt, angeordnet. Mittels der Medienzuführung wird ein Arbeitsmedium, insbesondere ein Antriebsfluid, von einer Medienquelle und/ oder einem Medienanschluss zu einer Aufnahmevorrichtung 6 für zumindest eine Reinigungs- und/ oder Pflegkapsel 7 und anschließend in das zumindest eine zu reinigende und/ oder pflegende Instrument 2 geleitet. Das Instrument 2 ist hierzu mittels einer Kupplungsvorrichtung lösbar mit dem Reinigungs- und/ oder Pflegegerät 1 verbunden. Ein Betätigungsmechanismus 12 an dem Reinigungs- und/ oder Pflegegerät 1 dient dazu, die in einer Aufnahmevorrichtung 6 aufnehmbare Reinigungs- und/ oder Pflegekapsel 7 mit dem Reinigungs- und/ oder Pflegemedium für das medizinische Instrument 2 mit der Medienzuführung zu koppeln, so dass die Aufnahmevorrichtung 6 bzw. die Reinigungs- und/ oder Pflegekapsel 7 in Fluidverbindung mit der Medienzuführung steht. Der Betätigungsmechanismus 12 ist hierbei bevorzugt durch ein erstes feststehendes Gehäuseteil 13 und durch ein zweites zu dem ersten Gehäuseteil 13 beweglich gelagerten Gehäuseteil 14 gebildet. Beide Gehäuseteile 13, 14 sind über einen Schwenkmechanismus 42 miteinander verbunden. Durch eine Schwenkbewegung des zweiten Gehäuseteils 14 erfolgt die Koppelung der Reinigungs- und/ oder Pflegekapsel 7 mit der Medienzuführung des Reinigungs- und/ oder Pflegegeräts 1. Die Reinigungs- und/ oder Pflegekapsel 7 ist dadurch von dem zumindest einen Arbeitsmedium der Medienquelle und/ oder des Medienanschlusses durchströmbar. Das Reinigungs- und/ oder Pflegemedium ist somit durch das Arbeitsmedium in das zu reinigende und/ oder pflegende Instrument 2 einbringbar. Hierdurch können dem medizinische Instrument 2 je nach Instrumententyp und/ oder Hersteller Reinigungs- und/ oder Pflegemedien mittels der zugehörigen Reinigungs- und/ oder Pflegekapsel 7 zugeführt werden. Die medizinischen Instrumente 2 sind somit in einfacher Weise reinigbar und/ oder pflegbar.

In Figur 2 ist eine schematische Darstellung, insbesondere ein Fluidplan, eines bevorzugtes Ausführungsbeispiel des Reinigungs- und/ oder Pflegegeräts 1 gezeigt. Hierbei ist die zumindest eine Medienzuführung 5 des Reinigungs- und/ oder Pflegegeräts 1 mit einer Medienquelle 3, insbesondere mit einem von dem Reinigungs- und/ oder Pflegegerät 1 bevorzugt lösbaren Fluidtank 3, verbunden, welcher das Antriebsfluid zur Verfügung stellt. Zum Fördern des Antriebsfluids von dem Fluidtank 3 in die mit der Medienzuführung 5 verbundene Aufnahmevorrichtung 6 für die Reinigungs- und/ oder Pflegekapsel 7 sowie in das medizinische Instrument 2 weist die Zuführung 5 bevorzugt zumindest eine Pumpen- und/ oder Kompressoreinheit 18 auf. Mittels eines Heizelements 17 ist das Antriebsfluid zusätzlich erwärmbar. An der Aufnahmevorrichtung 6 für die Reinigungs- und/ oder Pflegekapsel 7 ist die Kupplungsvorrichtung 4 zur lösbaren Verbindung des medizinischen, insbesondere zahnärztlichen, Instruments 2 mit dem Reinigungs- und/ oder Pflegegerät 1 angeordnet. Die Kupplungsvorrichtung 4 ist hierbei bevorzugt als standardisierte Schnittstelle mit einem Kupplungszapfen 48 ausgebildet.

Zur Steuerung des Medienflusses durch die Medienzuführung 5 weist die Zuführung 5 bevorzugt mehrere Ventile, insbesondere zumindest einen Ventilblock 36 sowie zumindest ein Rückschlagventil 37 auf. Des Weiteren sind in dem Reinigungs- und/ oder Pflegegerät 1 zur Überwachung des Medienflusses mehrere Sensoren, wie z.B. ein Füllstandssensor 34 und ein Durchflussmesser 35 angeordnet. Die Sensoren 34, 35 sind hierbei vorzugsweise mittels mehrerer Steuerleitungen 44 mit der zentralen Steuer- und/ oder Bedieneinheit 21 verbunden. Des Weiteren sind die Betriebsparamater des zumindest einen Heizelements 17 und der zumindest einen Pumpen- und/ oder Kompressoreinheit 18 bevorzugt abhängig von der in der Aufnahmevorrichtung 6 angeordneten Reinigungs- und/ oder Pflegekapsel 7 automatisch einstellbar. Hierzu weist die zumindest eine Aufnahmevorrichtung 6 eine Erkennungsvorrichtung 19 auf, welche ausgebildet ist ein an der zumindest einen Reinigungs- und/ oder Pflegekapsel 7 angebrachtes Identifizierungselement 20 zu erkennen. Die Steuer- und/ oder Bedieneinheit 21 identifiziert die Reinigungs- und/ oder Pflegekapsel 7 und sendet abhängig von der Art bzw. von dem Typ der Reinigungs- und/ oder Pflegekapsel 7, insbesondere abhängig von dem in der Kapsel 7 gespeicherten Reinigungs- und/ oder Pflegemedium, bevorzugt elektrische Signale an die mehreren Förder- und/ oder Heizelemente 17, 18 der Medienzuführung 5 zur Steuerung des Mediendurchflusses.

Bevorzugt weist das Reinigungs- und/ oder Pflegegerät 1 des Weiteren eine Belegungserkennung 38 auf. Die Steuer- und/ oder Bedieneinheit 21 erkennt mittels des Sensors 38, ob ein zu reinigendes und/ oder pflegendes Instrument 2 an dem Reinigungs- und/ oder Pflegegerät 1 angeschlossen ist. Ein Reinigungs- und/ oder Pflegeprozess wird somit nur gestartet, wenn ein Instrument 2 an der Kupplungsvorrichtung 4 angeordnet ist.

Der Medienfluss durch das Reinigungs- und/ oder Pflegegerät 1 gemäß Figur 2 und durch das medizinische Instrument 2 stellt sich wie folgt dar: Das Arbeitsmedium, insbesondere das Antriebsfluid, wird mittels einer Pumpen- und/ oder Kompressoreinheit 18 von der Medienquelle 3, durch die Medienzuführung 5 und durch die Aufnahmevorrichtung 6 für die Reinigungs- und/ oder Pflegekapsel 7, welche zumindest ein Reinigungs- und/ oder Pflegemedium aufweist, in die Kupplungsvorrichtung 4 für das zumindest eine medizinische, insbesondere zahnärztliche, Instrument 2 gefördert. Hierbei wird die in der Aufnahmevorrichtung 6 angeordnete Reinigungs- und/ oder Pflegekapsel 7 von dem Arbeitsmedium durchströmt und das darin gespeicherte Reinigungs- und/ oder Pflegemedium mit dem Arbeitsmedium durch die Kupplungsvorrichtung 4 in das zu reinigende und/ oder pflegende Instrument 2, insbesondere in dessen Getriebe- und/ oder Spraykanäle geleitet. Hierzu weist die Kupplungsvorrichtung 4 zu den mehreren Kanälen des Instruments 2 korrespondierende Medienleitungen auf. Nach der Durchdringung der bevorzugt mehreren zu reinigenden und/ oder pflegenden Kanäle des medizinischen Instruments 2, tritt das überschüssige Reinigungs- und/ oder Pflegemedium an dem freien Ende des Instruments 2 aus. Dieses wird bevorzugt in einer Schale 39 des Reinigungs- und/ oder Pflegegeräts 1 aufgefangen.

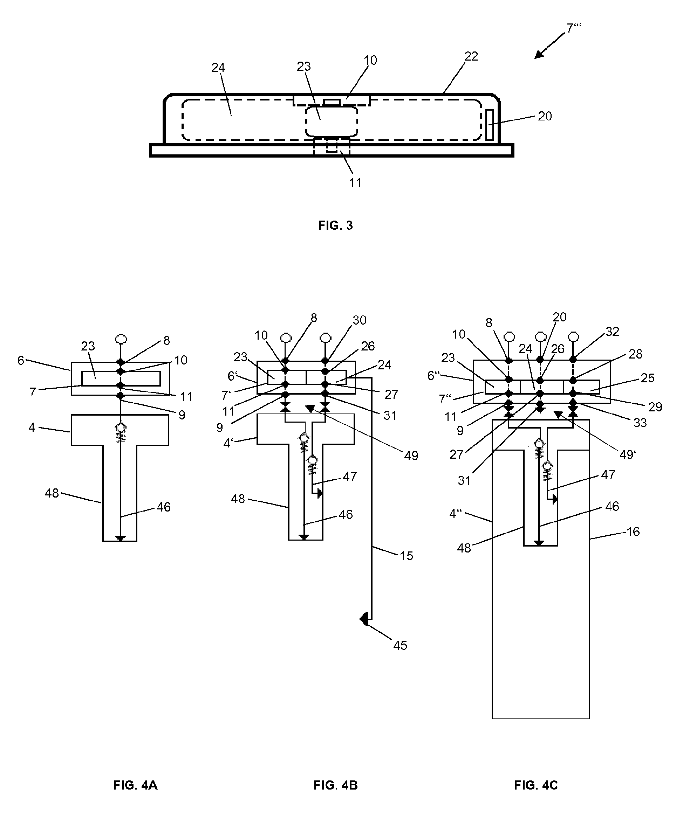

In Figur 3 ist ein Ausführungsbeispiel der Reinigungs- und/ oder Pflegekapsel 7'" in der Seitenansicht gezeigt. Hierbei ist die Reinigungs- und/ oder Pflegekapsel 7"'durch ein Gehäuse 22 mit einem Identifizierungselement 20, insbesondere einem Strichcode, zumindest einem versiegelten Medieneinlass 10 sowie zumindest einem versiegelten Medienauslass 11 gebildet. Die Versiegelungen sind bevorzugt durch zumindest zwei in der Aufnahmevorrichtung 6 angeordnete Stechvorrichtungen durchdringbar, so dass die zumindest eine in dem Gehäuse 22 der Kapsel 7'" angeordnete Kammer 23 mit dem zumindest einen Reinigungs- und/ oder Pflegemedium in Fluidverbindung mit der Medienzuführung 5 und dem Kupplungszapfen 4 bringbar ist. Durch die Beaufschlagung der Kammer 23 mit dem Antriebsfluid, kann schließlich das darin gespeicherte Reinigungs- und/ oder Pflegemedium bevorzugt in das zu reinigende und/ oder pflegende medizinische Instrument 2 transportiert werden. Die zumindest eine Kammer 23 für das zumindest eine Reinigungs- und/ oder Pflegemedium ist hierbei ausgebildet mindestens 0,05 Milliliter und maximal 5 Milliliter Reinigungs- und/ oder Pflegemedium zu speichern, so dass mittels der Reinigungs- und/ oder Pflegekapsel 7"'ein medizinisches Instrument 2 reinigbar und/ oder pflegbar ist.

Bevorzugt umfasst die Reinigungs- und/ oder Pflegekapsel 7'" eine zweite Kammer 24 für ein zweites Reinigungs- und/ oder Pflegemedium. Diese ist vorzugsweise derart ausgebildet, dass darin mindestens 20 Milliliter und maximal 100 Milliliter Reinigungs- und/ oder Pflegemedium gespeichert werden können. In dem Ausführungsbeispiel nach Figur 3 ist die erste Kammer 23 bevorzugt in der zweiten Kammer 24 angeordnet. Eine Membran zwischen beiden Kammern 23, 24 verhindert das Vermischen der zwei, vorzugsweise unterschiedlichen, Reinigungs- und/ oder Pflegemedien. Durch die Beaufschlagung der ersten Kammer 23 mit einem Antriebsfluid, vorzugsweise mit einem ersten Druckwert, kann das darin gespeicherte Reinigungs- und/ oder Pflegemedium aus der Reinigungs- und/ oder Pflegekapsel 7'" in die Medienzuführung 5 des Reinigungs- und/ oder Pflegegeräts 1 gefördert werden. Wird nun das Antriebsfluid mit einem zweiten Druckwert, welcher vorzugsweise den ersten Wert übersteigt, in die Reinigungs- und/ oder Pflegekapsel 7'" eingebracht, durchdringt dieses die vorzugsweise bei diesem Wert fluiddurchlässige Membran und das zweite Reinigungs- und/ oder Pflegemedium ist so aus der zweiten Kammer 24 in die Medienzuführung 5 des Reinigungs- und/ oder Pflegemediums förderbar.

Die Figuren 4A bis 4C zeigen drei Ausführungsbeispiele der Aufnahmevorrichtung 6, 6', 6" und der Kupplungsvorrichtung 4, 4', 4" für das Reinigungs- und/ oder Pflegegerät 1 sowie drei Ausführungsbeispiele der Reinigungs- und/ oder Pflegekapsel 7', 7", 7"'.

In Figur 4A ist das erste Ausführungsbeispiel der Kupplungsvorrichtung 4 zur Aufnahme des medizinischen Instruments 2, insbesondere zur Koppelung der in dem Instrument 2 angeordneten und zu reinigenden und/ oder pflegenden Kanäle mit dem Reinigungsgerät 1, gezeigt. Die Kupplungsvorrichtung 4 weist hierbei einen Kupplungszapfen 48, welcher bevorzugt lösbar von dem Reinigungsgerät 1 ausgebildet ist, sowie einen in dem Zapfen 48 angeordneten Medienkanal 46 auf. Der Medienkanal 46 tritt vorzugsweise stirnseitig an dem Kupplungszapfen 48 aus, so dass bevorzugt die Getriebekanäle des medizinischen Instruments 2 mit dem Reinigungsgerät 2 in Fluidverbindung bringbar sind. Des Weiteren ist der Medienkanal 46, welcher bevorzugt ein Rückschlagventil aufweist, mit der Medienzuführung 5 des Reinigungsgeräts 1, insbesondere mit der Aufnahmevorrichtung 6 für die Reinigungs- und/ oder Pflegekapsel 7, verbunden. Gemäß Figur 4A ist der Medienkanal 46 mit dem Medienauslass 9 der Aufnahmevorrichtung 6 gekoppelt. Das Antriebsfluid des Reinigungs- und/ oder Pflegegeräts 1 wird hierbei über einen Medieneinlass 8 in die Aufnahmevorrichtung 6 und mittels des Medienauslasses 9 durch den Kupplungszapfen 48 in das medizinische Instrument 2 geleitet. Durch die Aufnahme einer Reinigungs- und/ oder Pflegekapsel 7 in der Aufnahmevorrichtung 6, welche in diesem Ausführungsbeispiel eine einzige Kammer 23 für ein einziges Reinigungs- und/ oder Pflegemedium aufweist, ist das Reinigungs- und/ oder Pflegemedium mittels des Antriebsfluid bevorzugt in die Getriebekanäle des medizinischen Instruments dosierbar. Hierzu ist ein Medieneinlass 10 sowie ein Medienauslass 11 der Reinigungs- und/ oder Pflegekapsel 7 jeweils mit dem Medieneinlass 8 und dem Medienauslass 9 der Aufnahmevorrichtung 6 koppelbar. Das Antriebsfluid durchströmt hierbei die Reinigungs- und/ oder Pflegekapsel 7, insbesondere die Kammer 23.

In Figur 4B ist ein zweites Ausführungsbeispiel der Aufnahmevorrichtung 6' sowie der Kupplungsvorrichtung 4', insbesondere zur Reinigung und/ oder Pflege mehrerer, bevorzugt voneinander getrennter, Kanäle in dem medizinischen Instrument 2, abgebildet. Des Weiteren ermöglicht diese Ausführungsform neben der Reinigung und/ oder Pflege von innenliegenden Kanälen, insbesondere auch eine Außenreinigung des medizinischen Instruments 2. Hierzu ist die Aufnahmevorrichtung 6' mit zumindest einer Medienleitung 15, bevorzugt mit drei Medienleitungen, welche an ihrem freien Ende jeweils eine Düse 45 aufweisen, verbunden. Reinigungs- und/ oder Pflegemedien können so an der Außenseite des medizinischen Instruments 2 aufgebracht werden. Zur Reinigung und/ oder Pflege der mehreren Kanäle in dem medizinischen Instrument 2 weist der Kupplungszapfen 48 bevorzugt zwei Medienkanäle 46, 47 auf, welche jeweils mit einem Kanal des medizinischen Instruments 2 verbindbar sind. Über eine Schnittstelle 49 sind diese Kanäle 46, 47 wahlweise mit einem ersten Medienauslass 9 oder mit einem zweiten Medienauslass 31 der Aufnahmevorrichtung 6' koppelbar. Der Kupplungszapfen 48 ist hierzu vorzugsweise drehbar zu der Aufnahmevorrichtung 6' angeordnet. Bevorzugt wird der Zapfen 48 mittels eines Schwenkantriebs angetrieben. Alternativ ist es auch denkbar, dass die Aufnahmevorrichtung 6' zur Kupplungsvorrichtung 4' drehbar ausgebildet ist und mittels eines Antriebs angetrieben wird.

Das Antriebsfluid des Reinigungs- und/ oder Pflegegeräts wird in diesem Ausführungsbeispiel mittels zweier Medieneinlässe 8, 30 in die Aufnahmevorrichtung 6' geleitet. Hierdurch ist es möglich eine erste und zweite Kammer 23, 24 des zweiten Ausführungsbeispiels der Reinigungs- und/ oder Pflegekapsel 7' zu durchströmen, wodurch zwei Reinigungs- und/ oder Pflegemedien gleichzeitig oder nacheinander in das medizinische Instrument 2 einbringbar sind. Durch die zueinander drehbar ausgebildete Anordnung der Aufnahmevorrichtung 6' und der Kupplungsvorrichtung 4' können die zwei bevorzugt unterschiedlichen Reinigungs- und/ oder Pflegemedien zusätzlich in beide Kanäle 46, 47 des Kupplungszapfens 48 geleitet werden. Auch in diesem Ausführungsbeispiel sind die zwei Medieneinlässe 8, 30 sowie die Medienauslässe 9, 31 der Aufnahmevorrichtung 6' mit den Medieneinlässen 10, 26 und den Medienauslässen 11, 27 der Reinigungs- und/ oder Pflegekapsel 7' koppelbar.

Im Folgenden wird ein Ausführungsbeispiel eines Verfahrens zum Reinigen und/ oder Pflegen zumindest eines medizinischen, insbesondere zahnärztlichen, Instruments 2 mittels der in Figur 4B beschriebenen Ausführungsform der Aufnahmevorrichtung 6' für die Reinigungs- und/ oder Pflegekapsel 7" sowie der Kupplungsvorrichtung 4' des medizinischen Reinigungs- und/ oder Pflegegeräts 1 beschrieben. Die Reinigungs- und/ oder Pflegekapsel 7' weist hierbei zwei getrennte Kammern 23, 24 auf, wobei in einer ersten Kammer 23 ein Pflegemedium, wie zum Beispiel ein Pflegeöl, und in der zweiten Kammer 24 ein Reinigungsmittel gespeichert sind. Die Aufbereitung des medizinischen Instruments 2 ist bevorzugt durch folgende Schritte gekennzeichnet:
(1) Aufstecken des medizinischen Instruments 2 auf die Kupplungsvorrichtung 4' und Einlegen einer Reinigungs- und/ oder Pflegekapsel 7' in die Aufnahmevorrichtung 6',
(2) Leiten eines Antriebsfluids mittels der Medieneinlässe 26, 30 in die Kammer 24 der Kapsel 7' und Transportieren des Reinigungsmediums mittels der Medienauslässe 27, 31 aus der Kammer 24 durch den ersten Kanal 47 des Zapfen 48 und durch die Medienleitung 15, um einen Spraykanal des medizinische Instruments 2 sowie die Oberfläche des Instrument 2 zu reinigen,
(3) Automatisches oder manuelles Durchführen einer Drehbewegung der Kupplungsvorrichtung 4' um vorzugsweise 180 Grad,
(4) Erneutes Leiten des Antriebsfluids mittels der Medieneinlässe 26, 30 in die Kammer 24 der Kapsel 7' und Transportieren des Reinigungsmediums mittels der Medienauslässe 27, 31 aus der Kammer 24 durch den Kanal 46 des Zapfen 48, um einen Getriebekanal des medizinischen Instruments 2 zu reinigen,
(5) Automatisches oder manuelles Durchführen einer Drehbewegung der Kupplungsvorrichtung 4" um vorzugsweise 180 Grad,
(6) Leiten eines Antriebsfluids mittels der Medieneinlässe 8, 10 in die Kammer 23 der Kapsel 7' und Transportieren des Pflegemediums mittels der Medienauslässe 9, 11 aus der Kammer 23 durch den Kanal 46 des Kupplungszapfens 48, um den Getriebekanal des medizinischen Instruments 2 zu pflegen;

Figur 4C zeigt schließlich ein drittes Ausführungsbeispiel der Aufnahmevorrichtung 6", der Kupplungsvorrichtung 4", sowie der Reinigungs- und/ oder Pflegekapsel 7". Diese Ausführungsform ermöglicht neben eine Reinigung und/ oder Pflege insbesondere auch eine Desinfektion und/ oder Sterilisation des medizinischen Instruments 2. Hierzu weist die Kupplungsvorrichtung 4" bevorzugt ein Gehäuse 16 auf, welches das zumindest eine zu reinigende und/ oder pflegende Instrument 2, vorzugsweise druckdicht, umgibt. Das Gehäuse 16 ist hierbei bevorzugt lösbar, vorzugsweise mittels einer Schraubverbindung, von der Kupplungsvorrichtung 4" ausgebildet. Auch in diesem Ausführungsbeispiel umfasst die Kupplungsvorrichtung 4" einen Kupplungszapfen 48 mit bevorzugt zwei Medienkanälen 46, 47. Mittels der Medienübergabe 49' sind diese Kanäle 46, 47 wahlweise oder gleichzeitig mit dem ersten Medienauslass 9, dem zweiten Medienauslass 31 und/ oder dem dritten Medienauslass 33 der Aufnahmevorrichtung 6" koppelbar. Wie bereits in Figur 4B gezeigt, ist auch in diesem Ausführungsbeispiel die Kupplungsvorrichtung 4" drehbar zu der Aufnahmevorrichtung 6" angeordnet. Alternativ kann auch hier die Aufnahmevorrichtung 6" zur Kupplungsvorrichtung 4" drehbar ausgebildet sein.

Das Antriebsfluid des Reinigungs- und/ oder Pflegegeräts 1 wird in diesem Ausführungsbeispiel mittels dreier Medieneinlässe 8, 20, 32 der Aufnahmevorrichtung 6", insbesondere der mehreren Kammern 23, 24, 25 der Reinigungs- und/ oder Pflegekapsel 7", zugeführt. Hierzu sind die Medieneinlässe 8, 20, 32 sowie die Medienauslässe 9, 31, 33 der Aufnahmevorrichtung 6" mit den Medieneinlässen 10, 26, 28 und den Medienauslässen 11, 27, 29 der Reinigungs- und/ oder Pflegekapsel 7" koppelbar.

Folgend wird ein Ausführungsbeispiel eines Verfahrens zum Reinigen, Pflegen und Desinfizieren oder Sterilisieren zumindest eines medizinischen, insbesondere zahnärztlichen, Instruments 2 mittels der in Figur 4C beschriebenen Ausführungsform der Aufnahmevorrichtung 6", der Kupplungsvorrichtung 4" sowie der Reinigungs- und/ oder Pflegekapsel 7" beschrieben. Die Reinigungs- und/ oder Pflegekapsel 7" umfasst hierbei drei getrennte Kammern 23, 24, 25, welche ein Reinigungsmedium, ein Pflegemedium sowie ein Sterilisationsmittel beinhalten. Das Sterilisationsmittel umfasst hierbei bevorzugt demineralisiertes Wasser, welches mittels des Arbeitsmediums, insbesondere mittels Heißluft, in Wasserdampf umgewandelt wird. Die Aufbereitung des medizinischen Instruments 2 ist bevorzugt durch folgende Schritte gekennzeichnet:
(1) Aufstecken des medizinischen Instruments 2 auf die Kupplungsvorrichtung 4" und Einlegen einer Reinigungs- und/ oder Pflegekapsel 7" in die Aufnahmevorrichtung 6",
(2) Leiten eines Antriebsfluids mittels der Medieneinlässe 8, 10 in die Kammer 23 der Kapsel 7" und Transportieren des Reinigungsmediums mittels der Medienauslässe 9, 11 aus der Kammer 23 durch den Kanal 46 des Zapfen 48, um einen Getriebekanal des medizinische Instruments 2 zu reinigen,
(3) Automatisches oder manuelles Durchführen einer Drehbewegung der Kupplungsvorrichtung 4" um vorzugsweise 120 Grad,
(4) Erneutes Leiten des Antriebsfluids mittels der Medieneinlässe 8, 10 in die Kammer 23 der Kapsel 7" und Transportieren des Reinigungsmediums mittels der Medienauslässe 9, 11 aus der Kammer 23 durch den Kanal 47 des Zapfen 48, um einen Spraykanal des medizinischen Instruments 2 zu reinigen,
(5) Und gleichzeitiges oder anschließendes Leiten eines Antriebsfluids mittels der Medieneinlässe 20, 26 in die Kammer 24 der Kapsel 7" und Transportieren des Pflegemediums mittels der Medienauslässe 27, 31 aus der Kammer 24 durch den Kanal 46 des Kupplungszapfens 48, um den Getriebekanal des medizinischen Instruments 2 zu pflegen;
(6) Automatisches oder manuelles Durchführen einer Drehbewegung des Kupplungszapfens 48 um vorzugsweise 120 Grad,
(7) Leiten eines Antriebsfluids mittels der Medieneinlässe 28, 32 in die Kammer 25 der Kapsel 7" und Transportieren des Sterilisationsmediums, insbesondere des erzeugten Wasserdampfes, mittels der Medienauslässe 29, 33 aus der Kammer 25 durch den Kanal 46 des Zapfens 48, um den Getriebekanal des medizinische Instruments 2 zu sterilisieren,
(8) Automatisches oder manuelles Durchführen einer Drehbewegung des Kupplungszapfens 48 um vorzugsweise 120 Grad,
(9) Leiten eines Antriebsfluids mittels der Medieneinlässe 28, 32 in die Kammer 25 der Kapsel 7" und Transportieren des Wasserdampfes mittels der Medienauslässe 29, 33 aus der Kammer 25 durch den Kanal 47 des Zapfen 48 und durch den Spraykanal des medizinischen Instruments 2 in das Gehäuse 16 der Kupplungsvorrichtung 4", um den Spraykanal sowie die Oberflächen des medizinischen Instruments 2 zu sterilisieren;

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Reinigung- und/ oder Pflegegerät (1) für zumindest ein medizinisches, insbesondere zahnärztliches, Instrument (2) mit zumindest einer Medienquelle (3) und/ oder zumindest einem Medienanschluss zur Versorgung des Reinigungs- und/ oder Pflegegeräts (1) mit zumindest einem Arbeitsmedium, insbesondere mit einem Antriebsfluid, zumindest einer Kupplungsvorrichtung (4, 4', 4") zur lösbaren Verbindung des medizinischen, insbesondere zahnärztlichen, Instruments (2) mit dem Reinigungs- und/ oder Pflegegerät (1), sowie zumindest einer Medienzuführung (5), welche die zumindest eine Medienquelle (3) und/ oder den zumindest einen Medienanschluss mit dem zumindest einen zu reinigenden und/ oder pflegenden medizinischen Instrument (2) verbindet, **dadurch gekennzeichnet, dass** die zumindest eine Medienzuführung (5) zumindest eine Aufnahmevorrichtung (6, 6', 6") für zumindest eine Reinigungs- und/ oder Pflegekapsel (7, 7', 7") aufweist, wobei die Aufnahmevorrichtung (6, 6', 6") derart ausgebildet ist, dass die zumindest eine Reinigungs- und/ oder Pflegekapsel (7, 7', 7") von dem zumindest einen Arbeitsmedium der Medienquelle (3) und/ oder des Medienanschlusses durchströmbar ist, so dass das Arbeitsmedium mit dem Reinigungs- und/ oder Pflegemedium in das zu reinigende und/ oder pflegende Instrument (2) einbringbar ist.

2. Reinigung- und/ oder Pflegegerät (1) nach Anspruch 1, **dadurch gekennzeichnet, dass** die zumindest eine Aufnahmevorrichtung (6, 6', 6") der Medienzuführung (5) zumindest einen Medieneinlass (8) und zumindest einen Medienauslass (9) aufweist, welche mit zumindest einem Medieneinlass (10) und zumindest einem Medienauslass (11) der Reinigungs- und/ oder Pflegekapsel (7, 7', 7") koppelbar sind.

3. Reinigung- und/ oder Pflegegerät (1) nach Anspruch 2, **dadurch gekennzeichnet, dass** die zumindest eine Aufnahmevorrichtung (6, 6', 6") einen Betätigungsmechanismus (12) umfasst, um den zumindest einen Medieneinlass (10) und den zumindest einen Medienauslass (11) der Reinigungs- und/ oder Pflegekapsel (7, 7', 7") mit dem zumindest einen Medieneinlass (8) und dem zumindest einen Medienauslass (9) der Aufnahmevorrichtung (6, 6', 6") zu verbinden.

4. Reinigung- und/ oder Pflegegerät (1) nach Anspruch 3, **dadurch gekennzeichnet, dass** der Betätigungsmechanismus (12) durch ein erstes feststehendes Gehäuseteil (13) und durch ein zweites zu dem ersten Gehäuseteil (13) beweglich gelagerten Gehäuseteil (14) gebildet ist, wobei eines der Gehäuseteile (13, 14) den zumindest einen Medieneinlass (8) und das andere Gehäuseteil den zumindest einen Medienauslass (9) der Aufnahmevorrichtung (6, 6', 6") umfasst.

5. Reinigung- und/ oder Pflegegerät (1) nach einem der vorherigen Ansprüche, **dadurch gekennzeichnet, dass** die zumindest eine Aufnahmevorrichtung (6') mit zumindest einer Medienleitung (15) verbunden ist, welche derart in dem Reinigungs- und/ oder Pflegegerät (1) angeordnet ist, dass das Arbeitsmedium mit dem Reinigungs- und/ oder Pflegemedium auf der Außenseite des zu reinigenden und/ oder pflegenden Instruments (2) aufbringbar ist.

6. Reinigung- und/ oder Pflegegerät (1) nach einem der vorherigen Ansprüche, **dadurch gekennzeichnet, dass** die zumindest eine Kupplungsvorrichtung (4, 4', 4") lösbar von dem Reinigungs- und/ oder Pflegegerät (1) ausgebildet ist.

7. Reinigung- und/ oder Pflegegerät (1) nach Anspruch 6, **dadurch gekennzeichnet, dass** die zumindest eine Kupplungsvorrichtung (4") ein Gehäuse (16) umfasst, welches das zumindest eine zu reinigende und/ oder pflegende Instrument (2), vorzugsweise druckdicht, umgibt.

8. Reinigung- und/ oder Pflegegerät (1) nach einem der vorherigen Ansprüche, **dadurch gekennzeichnet, dass** die zumindest eine Aufnahmevorrichtung (6, 6', 6") und/ oder die zumindest eine Kupplungsvorrichtung (4, 4', 4") drehbar an dem Reinigungs- und/ oder Pflegegerät (1) angeordnet ist.

9. Reinigung- und/ oder Pflegegerät (1) nach einem der vorherigen Ansprüche, **dadurch gekennzeichnet, dass** die zumindest eine Medienzuführung (5) und/ oder die zumindest eine Aufnahmevorrichtung (6, 6', 6") zum Erwärmen des Arbeitsmediums und/ oder des Reinigungs- und/ oder Pflegemediums zumindest ein Heizelement (17) und/ oder zum Fördern und/ oder Verdichten des zumindest einen Arbeitsmediums und/ oder des zumindest einen Reinigungs- und/ oder Pflegemediums zumindest eine Pumpen- und/ oder Kompressoreinheit (18) aufweist.

10. Reinigung- und/ oder Pflegegerät (1) nach einem der vorherigen Ansprüche, **dadurch gekennzeichnet, dass** die zumindest eine Aufnahmevorrichtung (6) eine Erkennungsvorrichtung (19) aufweist, welche ausgebildet ist ein an der zumindest einen Reinigungs- und/ oder Pflegekapsel (7, 7') angebrachtes Identifizierungselement (20) zu erkennen, so dass die Betriebsparamater des zumindest einen Heizelements (19) und/ oder der zumindest einen Pumpen- und/ oder Kompressoreinheit (18) mittels einer Steuer- und/ oder Bedieneinheit (21) automatisch einstellbar sind.

11. Reinigungs- und/ oder Pflegekapsel (7, 7', 7") für ein Reinigungs- und/ oder Pflegegerät (1) nach einen der vorherigen Ansprüche, **gekennzeichnet durch** ein Gehäuse (22) mit zumindest einer Kammer (23) zur Aufbewahrung von zumindest einem Reinigungs- und/ oder Pflegemedium, zumindest einem versiegelten Medieneinlass (10) sowie zumindest einem versiegelten Medienauslass (11), wobei die zumindest eine Kammer (23) ausgebildet ist mindestens 0,05 Milliliter und maximal 5 Milliliter Reinigungs- und/ oder Pflegemedium zu speichern, so dass mittels der Reinigungs- und/ oder Pflegekapsel (7) ein medizinisches Instrument (2) reinigbar und/ oder pflegbar ist.

12. Reinigungs- und/ oder Pflegekapsel (7, 7', 7") nach Anspruch 11, **dadurch gekennzeichnet, dass** das Gehäuse (22) zumindest eine zweite Kammer (24) für ein zweites Reinigungs- und/ oder Pflegemedium umfasst, wobei die zweite Kammer (24) ausgebildet ist mindestens 20 Milliliter und maximal 100 Milliliter Reinigungs- und/ oder Pflegemedium zu speichern.

13. Reinigungs- und/ oder Pflegekapsel (7, 7', 7") nach Anspruch 11 oder 12, **dadurch gekennzeichnet, dass** das Gehäuse (22) ein Identifizierungselement (20), insbesondere einen Strichcode, zur Erkennung der Reinigungs- und/ oder Pflegekapsel (7, 7', 7") umfasst.

14. Verfahren zum Reinigen und/ oder Pflegen zumindest eines medizinischen, insbesondere zahnärztlichen, Instruments (2) mit einem Reinigungs- und/ oder Pflegegerät (1), wobei ein Arbeitsmedium, insbesondere ein Antriebsfluid, mittels einer Medienzuführung (5) von einer Medienquelle (3) und/ oder einem Medienanschluss durch eine Aufnahmevorrichtung (6, 6', 6") für eine Reinigungs- und/ oder Pflegekapsel (7, 7', 7"), welche zumindest ein Reinigungs- und/ oder Pflegemedium aufweist, in eine Kupplungsvorrichtung (4, 4', 4") für das zumindest eine medizinische, insbesondere zahnärztliche, Instrument (2) geleitet wird, **dadurch gekennzeichnet, dass** die in der Aufnahmevorrichtung (6, 6', 6") angeordnete Reinigungs- und/ oder Pflegekapsel (7, 7', 7") von dem Arbeitsmedium durchströmt wird und das Reinigungs- und/ oder Pflegemedium mit dem Arbeitsmedium in das zu reinigende und/ oder pflegende Instrument (2) geleitet wird.

15. Verfahren nach Anspruch 15, **dadurch gekennzeichnet, dass** das Arbeitsmedium und/ oder das Reinigungs- und/ oder Pflegemedium mittels eines Heizelements (17) erwärmt und/ oder mittels einer Pumpen- und/ oder Kompressoreinheit (18) verdichtet wird.

## Geänderte Patentansprüche

### Geänderte Patentansprüche gemäss Regel 137(2) EPÜ.

1. Reinigung- und/ oder Pflegegerät (1) für zumindest ein medizinisches, insbesondere zahnärztliches, Instrument (2) mit zumindest einer Medienquelle (3) und/ oder zumindest einem Medienanschluss zur Versorgung des Reinigungs- und/ oder Pflegegeräts (1) mit zumindest einem Arbeitsmedium, insbesondere mit einem Antriebsfluid, zumindest einer Kupplungsvorrichtung (4, 4', 4") zur lösbaren Verbindung des medizinischen, insbesondere zahnärztlichen, Instruments (2) mit dem Reinigungs- und/ oder Pflegegerät (1), sowie zumindest einer Medienzuführung (5), welche die zumindest eine Medienquelle (3) und/ oder den zumindest einen Medienanschluss mit dem zumindest einen zu reinigenden und/ oder pflegenden medizinischen Instrument (2) verbindet, **dadurch gekennzeichnet, dass** die zumindest eine Medienzuführung (5) zumindest eine Aufnahmevorrichtung (6, 6', 6") für zumindest eine Reinigungs- und/ oder Pflegekapsel (7, 7', 7") mit zumindest einem Reinigungs- und/ oder Pflegemedium aufweist, wobei die Aufnahmevorrichtung (6, 6', 6") derart ausgebildet ist, dass die zumindest eine Reinigungs- und/ oder Pflegekapsel (7, 7', 7") und das darin gespeicherte Reinigungs- und/ oder Pflegemedium von dem zumindest einen Arbeitsmedium der Medienquelle (3) und/ oder des Medienanschlusses durchströmbar ist, so dass das Arbeitsmedium mit dem Reinigungs- und/ oder Pflegemedium in das zu reinigende und/ oder pflegende Instrument (2) einbringbar ist.

2. Reinigung- und/ oder Pflegegerät (1) nach Anspruch 1, **dadurch gekennzeichnet, dass** die zumindest eine Aufnahmevorrichtung (6, 6', 6") der Medienzuführung (5) zumindest einen Medieneinlass (8) und zumindest einen Medienauslass (9) aufweist, welche mit zumindest einem Medieneinlass (10) und zumindest einem Medienauslass (11) der Reinigungs- und/ oder Pflegekapsel (7, 7', 7") koppelbar sind.

3. Reinigung- und/ oder Pflegegerät (1) nach Anspruch 2, **dadurch gekennzeichnet, dass** die zumindest eine Aufnahmevorrichtung (6, 6', 6") einen Betätigungsmechanismus (12) umfasst, um den zumindest einen Medieneinlass (10) und den zumindest einen Medienauslass (11) der Reinigungs- und/ oder Pflegekapsel (7, 7', 7") mit dem zumindest einen Medieneinlass (8) und dem zumindest einen Medienauslass (9) der Aufnahmevorrichtung (6, 6', 6") zu verbinden.

4. Reinigung- und/ oder Pflegegerät (1) nach Anspruch 3, **dadurch gekennzeichnet, dass** der Betätigungsmechanismus (12) durch ein erstes feststehendes Gehäuseteil (13) und durch ein zweites zu dem ersten Gehäuseteil (13) beweglich gelagerten Gehäuseteil (14) gebildet ist, wobei eines der Gehäuseteile (13, 14) den zumindest einen Medieneinlass (8) und das andere Gehäuseteil den zumindest einen Medienauslass (9) der Aufnahmevorrichtung (6, 6', 6") umfasst.

5. Reinigung- und/ oder Pflegegerät (1) nach einem der vorherigen Ansprüche, **dadurch gekennzeichnet, dass** die zumindest eine Aufnahmevorrichtung (6') mit zumindest einer Medienleitung (15) verbunden ist, welche derart in dem Reinigungs- und/ oder Pflegegerät (1) angeordnet ist, dass das Arbeitsmedium mit dem Reinigungs- und/ oder Pflegemedium auf der Außenseite des zu reinigenden und/ oder pflegenden Instruments (2) aufbringbar ist.

6. Reinigung- und/ oder Pflegegerät (1) nach einem der vorherigen Ansprüche, **dadurch gekennzeichnet, dass** die zumindest eine Kupplungsvorrichtung (4, 4', 4") lösbar von dem Reinigungs- und/ oder Pflegegerät (1) ausgebildet ist.

7. Reinigung- und/ oder Pflegegerät (1) nach Anspruch 6, **dadurch gekennzeichnet, dass** die zumindest eine Kupplungsvorrichtung (4") ein Gehäuse (16) umfasst, welches das zumindest eine zu reinigende und/ oder pflegende Instrument (2), vorzugsweise druckdicht, umgibt.

8. Reinigung- und/ oder Pflegegerät (1) nach einem der vorherigen Ansprüche, **dadurch gekennzeichnet, dass** die zumindest eine Aufnahmevorrichtung (6, 6', 6") und/ oder die zumindest eine Kupplungsvorrichtung (4, 4', 4") drehbar an dem Reinigungs- und/ oder Pflegegerät (1) angeordnet ist.

9. Reinigung- und/ oder Pflegegerät (1) nach einem der vorherigen Ansprüche, **dadurch gekennzeichnet, dass** die zumindest eine Medienzuführung (5) und/ oder die zumindest eine Aufnahmevorrichtung (6, 6', 6") zum Erwärmen des Arbeitsmediums und/ oder des Reinigungs- und/ oder Pflegemediums zumindest ein Heizelement (17) und/ oder zum Fördern und/ oder Verdichten des zumindest einen Arbeitsmediums und/ oder des zumindest einen Reinigungs- und/ oder Pflegemediums zumindest eine Pumpen- und/ oder Kompressoreinheit (18) aufweist.

10. Reinigung- und/ oder Pflegegerät (1) nach einem der vorherigen Ansprüche, **dadurch gekennzeichnet, dass** die zumindest eine Aufnahmevorrichtung (6) eine Erkennungsvorrichtung (19) aufweist, welche ausgebildet ist ein an der zumindest einen Reinigungs- und/ oder Pflegekapsel (7, 7') angebrachtes Identifizierungselement (20) zu erkennen, so dass die Betriebsparamater des zumindest einen Heizelements (19) und/ oder der zumindest einen Pumpen- und/ oder Kompressoreinheit (18) mittels einer Steuer- und/ oder Bedieneinheit (21) automatisch einstellbar sind.

11. Reinigungs- und/ oder Pflegekapsel (7, 7', 7") für ein Reinigungs- und/ oder Pflegegerät (1) nach einen der vorherigen Ansprüche, **gekennzeichnet durch** ein Gehäuse (22) mit zumindest einer Kammer (23) zur Aufbewahrung von zumindest einem Reinigungs- und/ oder Pflegemedium, zumindest einem versiegelten Medieneinlass (10) sowie zumindest einem versiegelten Medienauslass (11), wobei die zumindest eine Kammer (23) ausgebildet ist mindestens 0,05 Milliliter und maximal 5 Milliliter Reinigungs- und/ oder Pflegemedium zu speichern, so dass mittels der Reinigungs- und/ oder Pflegekapsel (7) ein medizinisches Instrument (2) reinigbar und/ oder pflegbar ist.

12. Reinigungs- und/ oder Pflegekapsel (7, 7', 7") nach Anspruch 11, **dadurch gekennzeichnet, dass** das Gehäuse (22) zumindest eine zweite Kammer (24) für ein zweites Reinigungs- und/ oder Pflegemedium umfasst, wobei die zweite Kammer (24) ausgebildet ist mindestens 20 Milliliter und maximal 100 Milliliter Reinigungs- und/ oder Pflegemedium zu speichern.

13. Reinigungs- und/ oder Pflegekapsel (7, 7', 7") nach Anspruch 11 oder 12, **dadurch gekennzeichnet, dass** das Gehäuse (22) ein Identifizierungselement (20), insbesondere einen Strichcode, zur Erkennung der Reinigungs- und/ oder Pflegekapsel (7, 7', 7") umfasst.

14. Verfahren zum Reinigen und/ oder Pflegen zumindest eines medizinischen, insbesondere zahnärztlichen, Instruments (2) mit einem Reinigungs- und/ oder Pflegegerät (1) und einer Reinigungs- und/ oder Pflegekapsel (7, 7', 7"), welche zumindest ein Reinigungs- und/ oder Pflegemedium aufweist, wobei ein Arbeitsmedium, insbesondere ein Antriebsfluid, mittels einer Medienzuführung (5) von einer Medienquelle (3) und/ oder einem Medienanschluss durch eine Aufnahmevorrichtung (6, 6', 6") für die Reinigungs- und/ oder Pflegekapsel (7, 7', 7") in eine Kupplungsvorrichtung (4, 4', 4") für das zumindest eine medizinische, insbesondere zahnärztliche, Instrument (2) geleitet wird, **dadurch gekennzeichnet, dass** die in der Aufnahmevorrichtung (6, 6', 6") angeordnete Reinigungs- und/ oder Pflegekapsel (7, 7', 7") und das darin gespeicherte Reinigungs- und/ oder Pflegemedium von dem Arbeitsmedium durchströmt wird und das Reinigungs- und/ oder Pflegemedium mit dem Arbeitsmedium in das zu reinigende und/ oder pflegende Instrument (2) geleitet wird.

15. Verfahren nach Anspruch 15, **dadurch gekennzeichnet, dass** das Arbeitsmedium und/ oder das Reinigungs- und/ oder Pflegemedium mittels eines Heizelements (17) erwärmt und/ oder mittels einer Pumpen- und/ oder Kompressoreinheit (18) verdichtet wird.
